# EUROPEAN PATENT APPLICATION

(11) **EP 2 161 040 A1**
(43) Date of publication of application: **10.03.2010**
(21) Application number: 08163416.4
(22) Date of filing: 01.09.2008
(51) Int. Cl.: A61L 2/10

(54) **Apparatus for sterilizing tube lumens**

(71) Applicant: Danmarks Tekniske Universitet, 2800 Kgs. Lyngby (DK)
(72) Inventor: Bak, Jimmy, 2670, Greve (DK)
(74) Representative: Wetke, Ellen

(57) **Abstract**

The invention relates to an assembly for sterilizing surfaces and lumens of a medical device with a light source. The assembly comprises a medical device having a lumen and a coupling portion, and a light source configured to emit light having a corresponding coupling portion, the light source can emit light that reduces the number of or removes micro organisms from lumen and/or surfaces of the medical device.

The invention comprises an assembly constituted by a medical device having a lumen and a connector part (10), and a light source configured to emit light having bactericidal effect which light source has a corresponding connector part (5). The connector part (10) of the medical device and the corresponding connector part (5) of the light source can be joined such that the light source is placed outside and in extension of the lumen before sterilization allowing the proximal edge and lumen portion of the medical device to be sterilized.

## Description

### The technical field

The invention relates to an assembly for sterilizing surfaces and lumens of a medical device with a light source. The assembly comprises a medical device having a lumen and a coupling portion, and a light source configured to emit light having a corresponding coupling portion, the light source can emit light that reduces the number of or removes micro organisms from lumen and/or surfaces of the medical device.

### Prior art

US 2008/0051736 discloses a medical device in the form of an indwelling catheter provided with a light source configured such that light is transmitted from the light source into the catheter shaft or lumen for sterilization purposes. The intention is to sterilize the whole lumen of the catheter which is difficult as this necessitates that the light is guided from the light source through the length of the lumen e.g. this might necessitate that the material which the catheter has been constructed of can transmit light.

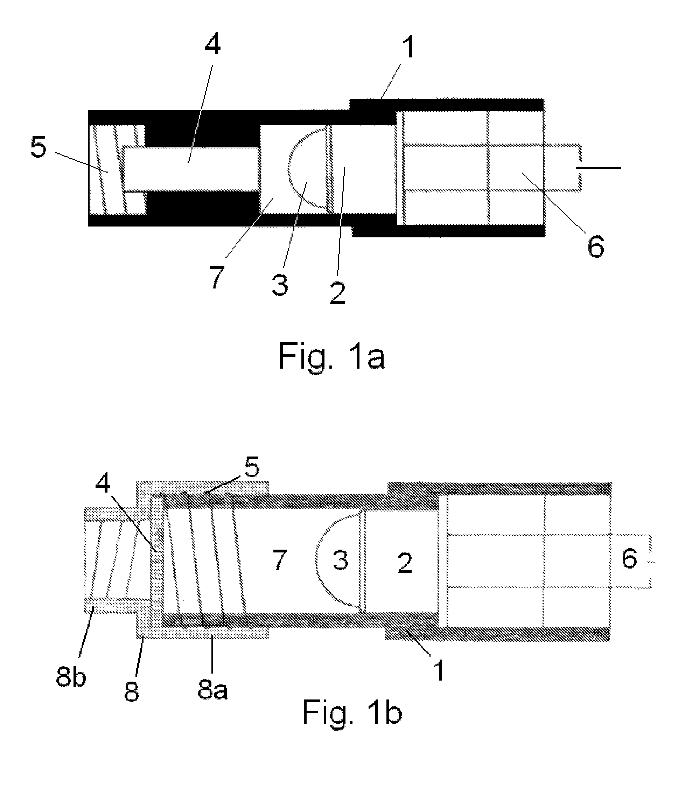

Further the light source as e.g. seen in fig. 1 A, 1 B and 1 C is integrated with the catheter into one assembly. This makes the assembly relatively expensive. Also it requires some dexterity and skills to correctly introduce or integrate the light source into the catheter and extract it i.e. this operation would be difficult for an elder patient. Furthermore, there is a risk of applying too much force during this process thereby breaking or compromising the light source.

The configuration of the light source is not actually described in this document and also it is not possible to control whether the light from the light source is actually emitted with the expected effect.

US 2008/0051736 implies that each catheter has its own light source constructed to fit the specific lumen. In contrast, the present invention employs one configured light source which can be coupled to different interfaces or coupling elements which are adapted to fit different catheters. This simplifies the method of sterilizing medical devices such as catheters and, as we will disclose, makes it simpler to couple the light source with the medical device.

### Summary of the invention

The object of the invention is to provide an assembly comprising:
- a medical device having a lumen and a connector part, and
- a light source configured to emit light having bactericidal effect which light source has a corresponding connector part,
- wherein the connector part of the medical device and the corresponding connector part of the light source can be joined such that the light source is placed outside and in extension of the lumen before sterilization allowing the proximal edge and lumen portion of the medical device to be sterilized.
   The medical device is normally an existing and/or commercial device.

According to one embodiment of the present invention the assembly further comprises a separate unit which is provided with a first coupling part and a second coupling part, where first coupling part of the separate unit can be joined to the corresponding connector part of the light source and the second coupling part which is fitted to the connector part of the medical devices. Normally the coupling can take place without the need for modification of existing medical devices.

According to one embodiment of the present invention the separate unit contains a sealing device such as a gasket or an O-bearing in order to prevent leakage of medical fluids from the medical device and into the surrounding or into the light source. The gasket or O-bearing could be loose and placed before use of the assembly or it could be a fixed part of the separate unit. The gasket can e.g. be made of silicone.

According to one embodiment of the present invention the medical device is a catheter having an ex-vivo portion.

According to one embodiment of the present invention the light source emits UVC light e.g. the light source comprises a light-emitting UVC-LED diode.

According to one embodiment of the present invention the light source comprises a ball or hemispherical lens system for optimal launch of the UVC light into the narrow tube openings of the medical device.

According to one embodiment of the present invention the light source comprises an UVC transparent optical window for separating the fluid in the medical device from the UVC LED diode and lens system. The windows can be made of UV grade quartz or CaF₂.

According to one embodiment of the present invention the light source has a length of max 50 mm and a diameter of max 15 mm.

According to one embodiment of the present invention the corresponding connector part is unthreaded, threaded, including luer, subminiature, press fit, and bayonet type couplings.

According to one embodiment of the present invention the medical device is an indwelling catheter e.g. selected from peripherally inserted central catheters, midline catheters, peripheral catheters, hemodialysis catheters and venous access ports or peripheral venous catheters and central venous access catheters.

According to one embodiment of the present invention the assembly is used for sterilizing the inlet portion of the lumen and surfaces of a medical device. The inlet portion is surfaces and lumen close to or part of the connector part of the medical device and the actual edge of the device which is normally placed ex-vivo during use of the medical device such as the proximal end of a catheter.

According to one embodiment of the present invention the assembly is used for partly sterilizing a medical device, where the light source has a sterilizing effect of the inlet portion of the lumen of a catheter, selected from peripheral venous catheters and central venous access catheters.

According to one embodiment of the present invention the assembly is used for partly sterilizing a medical device by joining
- a corresponding connector part of a light source with a connector part of a medical device in extension of a lumen in the medical device adjacent to the contact surface of the light source, and by filling
- the lumen of the medical device with liquid, and joining
- the light source to the medical device, wherein the light source emits light with bactericidal effect.

According to one embodiment of the present invention the assembly is used for partly sterilizing a medical device, wherein the refractive index n of the liquid in the lumen is higher than the refractive index of water, normally higher than the refractive index of the material of the medical device surrounding the lumen. This will increase the percentage of UVC light which can propagate towards the distal end of the lumen of the medical device.

According to one embodiment of the present invention the assembly is used for partly sterilizing a medical device, wherein the refractive index n of the liquid in the lumen is higher than 1.35, normally higher than 1.37, and/or the refractive index n of the material of the medical device is below 1.34, normally below 1.32.

According to one embodiment of the present invention the assembly is used for partly sterilizing a medical device, wherein the liquid in the lumen is a solution of NaCl or CaCl₂, and the material of the medical device surrounding the lumen is for instance polyurethane, silicone, polyethylene or teflon.

According to one embodiment of the present invention the assembly is used for partly sterilizing a medical device, wherein the necessary dosing time to obtain to obtain a disinfection rate of 99.99 % after preventive UVC light exposure is max 30 min.

According to one embodiment of the present invention the assembly is used for partly sterilizing a catheter, while the catheter is inserted in a subject.

According to one embodiment of the present invention provides a kit comprising a light source with a coupling portion, one or more separate units provided with different coupling parts corresponding to different standard catheters. Further the kit comprises a solution which can be poured into the lumen of the medical device providing a suitable refractive index for sterilizing the surface and lumen of the medical device e.g. catheters.

Embodiments of the invention will now be described with reference to the figures in which:
Figure 1 a and 1b each show an embodiment of a light source which can be part of the assembly according to the invention.
Figure 2 shows disinfection results of catheters using UVC light.
Figure 3 shows an assembly according to the invention comprising a catheter and a light source.
Figure 3a shows an enlargement of the coupling between the medical device and the light source of fig. 3.
Figure 4 shows an embodiment of an interface suitable for a catheter.

### Detailed description of the invention

The medical device will normally be a catheter, especially a catheter for indwelling use. In medicine a catheter is a tube that can be inserted into a body cavity, duct or vessel. Catheters thereby allow drainage, injection of fluids, diagnostic agents and/or medicine or access by surgical instruments. In most uses a catheter is a thin, flexible tube i.e. a "soft" catheter; in some uses, it is a larger, solid tube i.e. a "hard" catheter.

Intravascular catheters are indispensable in modern-day medical practice, particularly in intensive care units (ICUs). Catheter-related bloodstream infections (CRBSI) resulting from bacterial colonisation of an intravascular catheter are a significant clinical problem, magnified in recent years by the increasing use of intravascular catheters in intensive care, chemotherapy and total parental nutrition (TPN). In particular, central venous catheter-related infections are a common cause of bacteraemia and sepsis.

There are three routes by which infection can occur:
- Intraluminal
- Extraluminal
- Haematogenous

Most in-dwelling e.g. vascular catheters are colonized by micro-organisms. The colonizing micro-organisms are usually imbedded in a biofilm layer, they are metabolically active and viable, and they can already be present 24 h after insertion of the catheter.

Organisms causing bloodstream infections generally enter the bloodstream from the skin insertion site or through the connecting hub of the catheter which remains outside the skin but haematogenous seeding and contamination of infused fluids are possible causes as well.

When following the extraluminal route skin organisms migrate from the skin insertion site along the external surface of the catheter, colonizing the distal intravascular tip of the catheter and ultimately causing bloodstream infection.

When following the intraluminal route, organisms may be introduced into the hub e.g. by the hands of medical personnel. The subsequent colonization of the internal surface of the catheter may also cause bloodstream infection.

Many clinicians feel reluctant to remove the catheter, because most patients with cuffed tunnelled catheters have exhausted all other options for vascular access. The present invention is primarily directed to prevent intraluminal routed infections and the purpose of the invention is to prevent the formation of biofilm rather than removing the biofilm when it has been formed on the internal surfaces of the medical device.

Health-care institutions purchase millions of intravascular catheters each year.
The incidence of CRBSI varies considerably by type of catheter, frequency of catheter manipulation, and patient-related factors (e.g., underlying disease and acuity of illness). Peripheral venous catheters are the devices most frequently used for vascular access. Although the incidence of local or bloodstream infections associated with peripheral venous catheters is usually low, serious infectious complications produce considerable annual morbidity because of the frequency with which such catheters are used. However, the majority of serious catheter-related infections are associated with central venous catheters (CVCs), especially those that are placed in patients in ICUs.

In the ICU, central venous access might be needed for extended periods of time; patients can be colonized with hospital-acquired organisms; and the catheter can be manipulated multiple times per day for the administration of fluids, drugs, and blood products. Moreover, some catheters can be inserted in urgent situations, during which optimal attention to aseptic technique might not be feasible. Certain catheters (e.g., pulmonary artery catheters and peripheral arterial catheters) can be accessed multiple times per day for hemodynamic measurements or to obtain samples for laboratory analysis, augmenting the potential for contamination and subsequent clinical infection.

Specific examples of catheters are so-called peripherally inserted central catheters (PICCs), midline catheters, and peripheral catheters. A typical PICC, midline, or peripheral catheter contains a thin, flexible shaft, which contains one or more lumens and which terminates at the proximal end with a suitable fitting, such as a hub or other fitting. The primary difference between these three devices is the length of the tubing, with the peripheral catheter being the shortest and the PICC being the longest. The rationale for different lengths is driven by the type and duration of the therapy a patient is to receive.

Hemodialysis catheters are another important class of central venous access catheters. Hemodialysis catheters are commonly multi-lumen catheters in which one lumen is used to carry blood from the body to a dialysis machine, and another lumen returns blood to the body. Central venous access may be attained by puncture of various major blood vessels, including the internal jugular vein, subclavian vein, or femoral vein.

A catheter may further include various accessory components, for example, molded components, over-molded sub-assemblies, connecting fittings such as hubs, extension tubes, and so forth. Various catheter tips designs are known, including stepped tips, tapered tips, over-molded tips and split tips (for multilumen catheters), among others.

It is known that microorganisms, such as viruses, bacteria, fungi, protozoa, algae, and so forth can be inactivated (i.e., either killed or prevented from reproducing, e.g., by molecular rearrangement of the microorganisms DNA) using light of various wavelengths, including ultraviolet light of various wavelengths such as ultraviolet-C (UVC) light having a wavelength of 100 to 280 nm, ultraviolet-B (UVB) light having a wavelength 280 to 320 nm, and ultraviolet-A (UVA) light having a wavelength of 320 to 400 nm. For example, UVC light has a very short wavelength and kills bacteria and viruses so well that it is often used to sterilize surfaces. UVB light has also been reported to kill microorganisms.

Medical devices such as endoscopes commonly employ light emitting components, such as light sources and light guides, for introducing light into the subject and various coupling designs are available, which readily allow the connection and disconnection of light emitting components to and from the device. For example, couplers and end fittings for optical cables, which allow for efficient coupling of light to and from the optical cables, are presently known in the medical arts including those available from Codman, Fuji, Pentax, Pilling, Storz, and Wolf, among others. Of course other designs, including other unthreaded and threaded designs, including luer, subminiature, press fit, and bayonet type couplings, among others, may be employed.

The medical devices of the present invention comprise a shaft that contains one or more lumens (e.g., a tube, multilumen extrusion, etc.), which is introduced into a patient for either short or long term residency.

These and other aspects, embodiments and advantages of the present invention will become immediately apparent to those of ordinary skill in the art upon reading the disclosure to follow.

Fig. 1a and 1b shows embodiments of lights sources which can be used in connection with the present invention.

The light source shown in fig. 1 a comprises a housing 1, an UV source e.g. an UVC LED (Light Emitting Diode) having a socket 2 and an optical lens 3 e.g. flat, ball or hemispherical, an optical window 4 which guides the light to the catheter inlet and protect the entrance to the light source, a first connector part 5 comprising an inward thread i.e. female part which can connect to a medical device, and an electrical switch part 6 connecting to an electrical power supply.

The housing 1 provides a watertight encapsulation for the internal parts. The housing 1 has relatively small dimensions i.e. a length less than 10 cm, usually less than 5 cm, and a diameter less than 2 cm, usually less than 1.5 cm. The housing 1 is usually made of a hard polymer or plastic material (for instance acrylic plastic) or thin metal (for instance alumina) which makes it light, strong and easy to form and manufacture. The housing 1 is at the distal end i.e. the end facing the medical device equipped with a first connector part 5 which connector part 5 makes it possible either to link the light source directly to the medical device via a standard opening in the medical device, or to link the light source to an interface (not shown) which interface enables a link between the specific medical device and the light source. According to the shown embodiment the first connector part 5 is formed as a female thread which will fit into a corresponding male thread on a medical device for instance Luer conical fitting system or an interface.

The UV source used according to this embodiment comprises a diode equipped with a socket and a ball or hemispherical lens 3. The UV source emits light in the range between 200 and 300 nm which has a bactericidal effect. The space 7 between the lens/diode system 2, 3 and optical window 4 is occupied by air through which the UV light passes on the way to the optical window 4. The optical window 4 allows the UV light to pass into the openings of the tubing of the medical device (inner diameters of the tubes are typically in the range 1-5 mm) which is linked to the light source, and additionally the optical window 4 provides a watertight barrier which prevents liquid from the medical device (typically a high refractive index liquid filled into a catheter lumen) to enter the internal parts of the light source or eventually leak to the surroundings. When applying this light source it is e.g. not necessary to use optical fibers as the light source can be fastened directly to the medical device.

During the UVC disinfection procedure liquids inside the medical device are in close contact with the surfaces of the housing 1 and the surfaces of the optical window 4. Therefore it is necessary to be able to clean these surfaces in order to remove residues that otherwise attenuates the UVC light and thereby reduce the efficiency of the light source as the light source normally is used several times i.e. it might be used several times with one medical device and it might be used with several medical devices. The cleaning can be performed with liquid soap, alcohol such as isopropanol or ethanol or another solvent.

The UV source is electrically powered typically a few volts e.g. 6 V through a connection with the electrical switch part 6 which can be connected to a power supply with electrical cords. The power supply can either be placed at a distance or it can comprise batteries placed in connection with i.e. joined directly to the light source.

The embodiment shown in fig. 1b comprises a light source having the same or corresponding components as the embodiment of fig. 1a. Components with same or similar function are provided with the same reference number for the different embodiments disclosed in the present application.

Like the light source shown in fig. 1a the embodiment of fig. 1b comprises a housing 1, an UV source having a socket 2 and an optical lens 3 e.g. flat, ball or hemispherical, an optical window 4 which guides the light to the catheter inlet, a first connector part 5 which can connect to a medical device, and an electrical switch part 6 connecting to an electrical power supply.

The embodiment of fig. 1b is further provided with a separate unit 8 in the form of an interface having both a female part 8a with an inward thread which can be connected to the light source and a female part 8b with an inward thread having a smaller diameter and which can be connected to a medical device.

The optical window 4 of this embodiment has the same diameter as the opening of the light source. When the optical window 4 is provided with proper sealing it can be placed between the interface and the light source simply by turning the two units toward each other. This will make it easy to separate the units and clean them individually.

The optical window 4 can be made of UV grade quartz or CaF₂ and will normally be 1-2 mm thick.

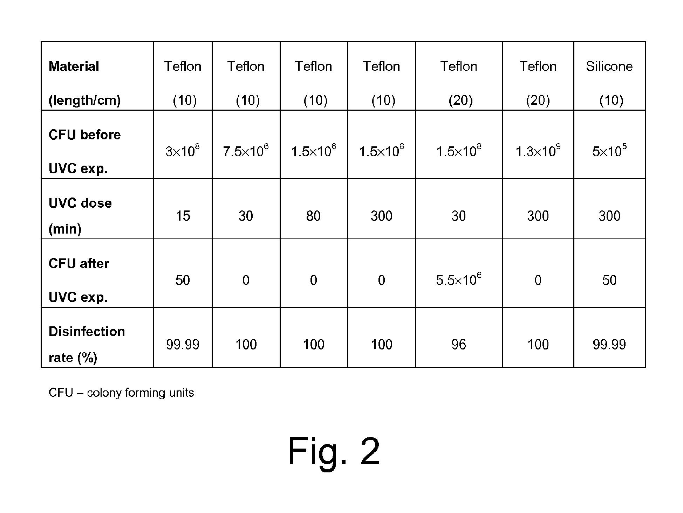

Fig. 2 shows a table comprising test results demonstrating the disinfection method on biofilm contaminated tube samples.

Tubes made in Teflon and silicones were cut in different lengths and were afterwards contaminated with biofilm. Two tube samples were cut pair-wize to the desired lengths i.e. 10 or 20 cm and connected to a flow system including a container with viable *Pseudomonas aeruginosa* and a peristaltic pump system. The tubes have an internal diameter 4 mm and an external diameter of 6 mm. The two pieces of tubes were filled with the bacteria contaminated aqueous solution and kept at 37°C during three days. In order to enhance the amount of biofilm at the inner surface of the tubes the solution was pumped continuously through the two tube samples placed in parallel two hours per day.

The tubes were removed after three days, one was used for control i.e. count of viable bacteria (Colony Forming Units CFU/ml) before UVC treatment and the other was UVC treated with the light source which in this case is a LED disinfection unit.. A modified version of the light source shown in fig. 1a was used in the experiments. The optical UV grade quartz window 4 was mounted i.e. pressed gently into the opening of the tubes before UVC exposure was initiated. The tube for treatment was filled with a 20 % saline solution (NaCl) before the UVC light was turned on.

It is well known that Teflon such as AF Teflon from Dupont has a very low refractive index 1.30 in the visible spectral region compared to other polymers. It is possible to obtain a relative high refractive index in the lumen of the tubes by injecting aqueous solutions with high concentrations of for instance sodium chloride or calcium chloride. If the refractive index of the liquid is higher than that of the tube material, light transmission through the tube is possible by internal reflections due to the liquid light guide principle.

Biofilm samples for viable count determination of both control and UVC treated samples were taken by brushing the entire inner surface (total length). Both samples were suspended in enriched broth solution. The viable count of the control and UVC treated tube samples can be seen in fig. 2. The level of biofilm contamination in the tubes before exposure to UVC light is seen to be very high. It is expected that the contamination level created here is much higher than that observed in newly inserted catheters.

The UVC disinfection unit was also tested using a 10 cm piece of silicone tube (Peritoneal catheter). The silicone tube used in this experiment had a refractory index of approximately 1.45. A disinfection rate of 99.99 % was achieved on this relatively high refractive index catheter tube material after 3 hours of exposure with the light source in form of a UVC LED unit.

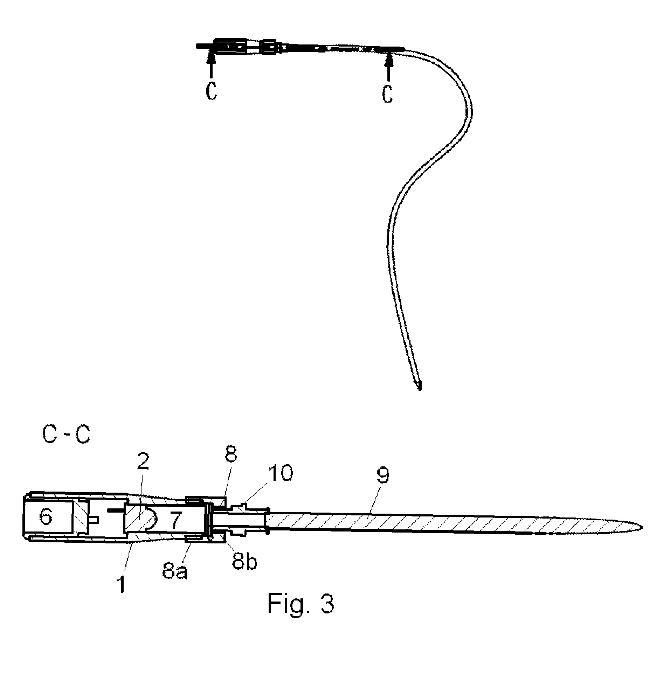

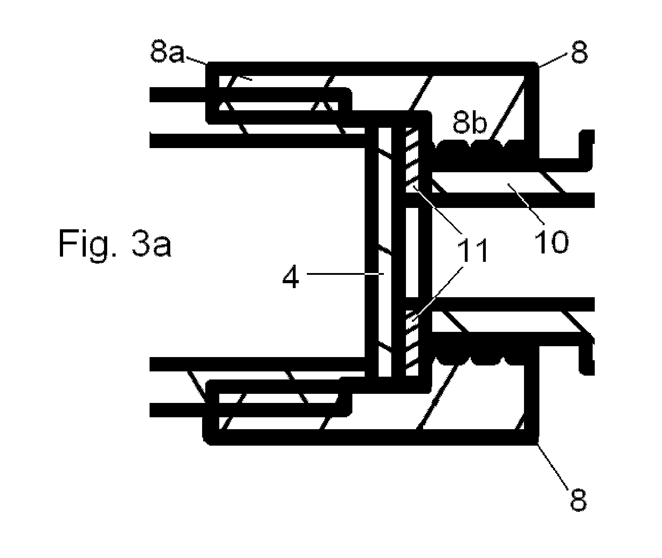

Figure 3 shows an assembly according to the invention comprising a light source and a separate unit 8 linked to a catheter 9 having an inlet hub 10 and an inner lumen for sterilization of the catheter inlet hub 10 and at least part of the inner lumen. In this embodiment the light source is of the same type as the light source shown in fig. 1 b.

Firstly, the separate unit 8 is assembled with the light source. The light source is screwed on the separate until watertight contact is made between a gasket 11 placed in the bottom of the separate unit and the light source. Fig. 3a shows an enlargement of the separate unit 8 used in fig. 3, and therefore the gasket 11 which is placed between the optical window 4 and the separate unit 8 becomes visible in fig. 3a. A watertight contact is made between the optical window 4 and the gasket 11 while light can flow through the optical window 4 and through the corresponding central opening of the gasket 11. After the separate unit 8 has been fastened to the light source the separate unit 8 is then screwed on the catheter inlet hub 10. Fig. 3 shows an assembly in which the inlet hub 10 of the catheter is equipped with a Luer fitting. The edge of the inlet hub 10 is screwed on the separate unit 8 until contact is made to the gasket 11. This ensures that an appropriate sealing of the catheter lumen is achieved.

After the light source has been assembled to the catheter the UVC treatment can be initiated. Before joining the assembly, the catheter has been filled with an aqueous solution of for instance sodium chloride for optimal guidance of the light through the inlet hub 10 and tube lumen. The UV LED diode 2 is powered by a low DC voltage source, e.g. 6 V. Typically a few hundreds microwatts UVC light is emitted from such a diode. The optimal wavelength used for bactericidal purposes is that close to 265 nm. An optical path length (1-2 cm) between the diode and edge of the inlet hub 10 ensures that the inner lumen of the inlet is exposed to UVC light. As illustrated in fig. 2 even a high level of contamination can be reduced substantially if the dose is sufficiently high and/or the catheter material has a low refractive index. Disinfection of a catheter made in materials which are normally used in the clinic, such as silicone (peritoneal catheter) are also possibly even if these tubes are heavily contaminated, see fig. 2.

The main purpose of the invention is not to sterilize or disinfect lumens or surfaces of medical devices often in the form of implants that are severely contaminated due to a very long dwell time. The intention is to use the invention as a preventative mean i.e. by sterilizing the inner portion of the catheter hub and outermost parts of the tube lumens. Therefore preventative UVC treatments of catheters with the described invention will normally takes place right after the medical implants have been inserted, repeated before and after use of the catheter for medical purposes e.g. before and after a hemodialysis session. No contamination of the inner surface of newly inserted catheters is expected.
During handling of the catheters i.e. opening and closing of inlet hubs by personnel, contact to the patient's skin and exposure from external possible sources of bacteria, such as medical equipment e.g. syringes and tubes, bacteria can penetrate through the inlet hub and start colonizing the inner lumen.

Therefore, it is important to sterilize as much of the proximal end of the catheter soon after it has been used in order to prevent biofilm formation, which eventually later on could be spread to the distal ends of the catheter.
The doses required for preventively disinfecting medical devices such as catheters are expected to be relatively low compared to the doses shown in fig. 2. The exact doses can be found by clinical trials in which catheters in-vivo are UVC treated, or alternatively can the number of CFU be determined to different dwell times while it is inside the patient.

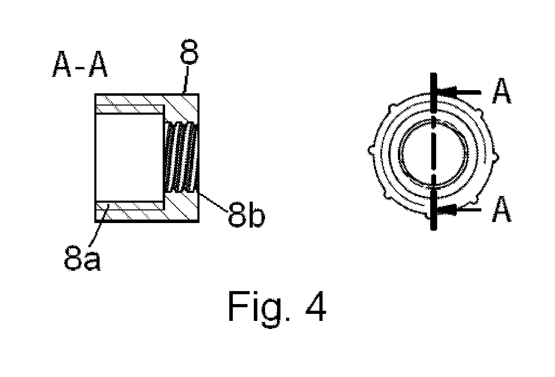

Fig. 4 shows a separate unit in the form of an interface having two oppositely placed coupling positions. A first coupling position 8b is to be coupled to a connector part 5 of a light source and a second coupling position 8a is to be coupled to a connector part on a medical device.

## Claims

1. An assembly comprising
- a medical device having a lumen and a connector part (10), and
- a light source configured to emit light having bactericidal effect which light source has a corresponding connector part (5),
- wherein the connector part (10) of the medical device and the corresponding connector part (5) of the light source can be joined such that the light source is placed outside and in extension of the lumen before sterilization allowing the proximal edge and lumen portion of the medical device to be sterilized.

2. An assembly according to claim 1, wherein further comprising a separate unit (8) which separate unit (8) is provided with a first coupling part and a second coupling part, where first coupling part of the separate unit can be joined to the corresponding connector part (5) of the light source and the second coupling part can be joined to the connector part (10) of the medical device.

3. An assembly according to claim 2 or 3, wherein the separate unit contains a sealing device such as a gasket or a O-bearing in order to prevent leakage of medical fluids from the medical device and into the surrounding or into the light source.

4. An assembly according to any of the claims 1-3, wherein the medical device is a catheter having an ex-vivo portion.

5. An assembly according to any of the claims 1-4, wherein the light source emits UVC light.

6. An assembly according to claim 5, wherein the light source comprises a light-emitting UVC-LED diode (2).

7. An assembly according to claim 6, wherein the light source comprises a ball or hemispherical lens system (3) for optimal launch of the UVC light into the narrow tube openings of the medical device.

8. An assembly according to claim 7, wherein the light source comprises an UVC transparent optical window (4) for separating the fluid in the medical device from the UVC LED diode and lens system.

9. An assembly according to any of the claims 6-8, wherein the light source has a length of max 50 mm and a diameter of max 15 mm.

10. An assembly according to any of the claims 1-9, wherein said corresponding connector part (5) is unthreaded, threaded, including luer, subminiature, press fit, and bayonet type couplings.

11. An assembly according to any of the claims 1-10, wherein the medical device is an indwelling catheter e.g. selected from peripherally inserted central catheters, midline catheters, peripheral catheters, hemodialysis catheters and venous access ports or peripheral venous catheters and central venous access catheters.

12. Use of the assembly according to the preceding claims for sterilizing of the inlet portion of the lumen and surfaces of a medical device.

13. A method for partly sterilizing a medical device
- a corresponding connector part (5) of a light source is joined to a connector part (10) of a medical device in extension of a lumen in the medical device adjacent to the contact surface of the light source,
- the lumen of the medical device is filled with liquid,
- after joining the light source to the medical device the light source emits light having bactericidal effect.

14. A method according to claim 13, wherein the refractive index n of the liquid in the lumen is higher than the refractive index of water, normally higher than the refractive index of the material of the medical device surrounding the lumen.

15. A method according to claim 13 or 14, wherein the refractive index n of the liquid in the lumen is higher than 1.35, normally higher than 1.37, and/or the refractive index n of the material of the medical device is below 1.34, normally below 1.32.

16. A method according to claim 13, wherein the liquid in the lumen is a solution of NaCl or CaCl₂, and the material of the medical device surrounding the lumen is e.g. polyurethane, silicone, polyethylene or Teflon

17. A method according to claim 13, wherein the necessary dosing time to obtain a disinfection rate of 99.99 % after preventive UVC light exposure is max 30.
